# EUROPEAN PATENT APPLICATION

(11) **EP 1 739 096 A1**
(43) Date of publication of application: **03.01.2007**
(21) Application number: 05719325.2
(22) Date of filing: 21.02.2005
(51) Int. Cl.: C08B 37/06, A61K 8/73, C12P 19/04

(54) **PECTIN ORIGINATING IN PLANT CELL**

(30) Priority: 24.03.2004 JP 2004087499
(71) Applicant: Mitsui Chemicals, Inc., Tokyo 105-7117 (JP)
(72) Inventor: KATO, Yoshihiro, c/o Mitsui Chemicals, Inc., Yamaguchi 7400061 (JP); MATSUBARA, Kouichi, c/o Mitsui Chemicals, Inc., Yamaguchi 7400061 (JP); TABATA, Homare, c/o Mitsui Chemicals, Inc., Chiba 2970017 (JP)
(74) Representative: Stuart, Ian Alexander
(86) International application number: PCT/JP2005/002706
(87) International publication number: WO 2005/090410

(57) **Abstract**

It is intended to find characteristics of a pectin having a high molecular weight as compared with the existing pectins which are obtained by extracting plants and provide a method whereby the pectin can be supplied in a large amount. A callus derived from a plant is cultured so as to produce a pectin having a high molecular weight in the culture or the culture broth. Then the desired pectin is obtained by separating from the thus obtained culture or culture broth and purifying. The pectin thus obtained of the present invention has favorable properties as a moisturizer and, therefore, is excellent as a cosmetic material.

## Description

### TECHNICAL FIELD

The present invention relates to a pectin obtained from a cell culture, and a cosmetic material containing the pectin. More specifically, the invention relates to a pectin obtained from a cell culture of a Nigella plant and a pectin having a high molecular weight used as a moisturizer.

### BACKGROUND ART

Polysaccharide called a pectic substance is contained in each organ of many plants, and exists mostly in meristem tissue or parenchyma tissue. The pectic substance refers to colloidal carbohydrates obtained in a plant or from a plant, comprises lots of galacturonic acid groups, and is a group of substances with the galacturonic groups bonded in a chain. Pectin exists in a form of protopectin insoluble in water in combination of cellulose, hemicellulose, lignin, protein, mineral and the like as a cell wall component of a plant. A substance which is soluble in a pectic substance and gelated with sugar and acid under an appropriate condition is called a pectin.

A method for preparing a pectin generally comprises (1) extraction under high temperature and acidity from a vegetable raw material, (2) purification of a liquid extract, and (3) a separating step of a pectin extracted from the liquid. Of these steps, at the step of acid extraction, plant materials are treated using a diluted acid such as nitric acid, sulfuric acid, hydrochloric acid or other inorganic or organic acid, usually, under the conditions of pH of from 1 to 2 and a temperature of from 80°C to 100°C. As the vegetable raw material which is usually used, citrus peel obtained by the preparation of juice and squeezed apple obtained by the preparation of an apple juice or an apple cider are used. However, the molecular weight of the pectin obtained from the vegetable raw material is about 6 x 10⁴ to 2 x 10⁵.

The pectin is known to be obtained by culturing a plant cell in a liquid medium. In Proc. Natl. Acad. Sci. USA, Vol. 85, pp. 2618 to 2622 (1988), there has been reported that a pectin having a molecular weight of 1.8 x 10⁴ was discharged into a liquid from a soybean culture cell, whereas its production amount was a little, i.e., 13.4 mg/l. In addition, a woody plant such as the above citrus or the like contains a large amount of a phenolic substance in a cell wall and has a hard tissue so that tissue culture is difficult. Therefore, the woody plant is not suitable for the production of a pectin. For that reason, plants capable of producing pectins in a large amount by culture have not been known.

In WO 9636693, there has been disclosed that many plant cells were stimulated by a DNA methylation inhibitor and pectin pieces were obtained. However, in the patent document, there has not been disclosed about either the molecular weight or the production amount of the resulting pectin.

The pectin has a variety of characteristics depending on the difference in the degrees of esterification or in the molecular weights, and has been mostly used in the food fields as a gelling agent, a thickening agent, a stabilizer and the like. The degree of esterification (DE) illustrating the degree of esterification refers to a value (%) showing a ratio of the esterified galacturonic acid (carboxyl group) occupied in the total galacturonic acids (total carboxyl groups). Pectins are classified into a high methoxyl pectin (HM pectin) and a low methoxyl pectin, depending on the fluctuation of the methyl-esterification degree. Usually, a pectin with DE of more than 50% is called a HM pectin, while a pectin with DE of 50% or less is called an LM pectin. In general, the greater part of the pectins extracted from the vegetable raw materials under a condition of high temperature and acidity as described above are HM pectins. In order to obtain LM pectins, it needs to further use acid, alkali, enzyme or ammonia for demethylation. When extracting pectins from the citrus peel, there has been a drawback such that sugar chains were broken at the same time with hydrolysis of ester due to the condition of high temperature and acidity or the demethylation treatment at the time of extraction, resulting in the reduction of the molecular weight. As a result, it is expected that a pectin having a high molecular weight has various properties, but there has not been known any method for stably producing such pectins.

It is already known that a pectin is used as one ingredient of a cosmetic material. A pectin used for a cosmetic material has only a molecular weight of from 6 x 10⁴ to 2 x 10⁵ and is just added as a gelling agent, a thickening agent, a stabilizer, and as an auxiliary agent of the effective cosmetic ingredient. But, it has not been known that pectins are used as an effective ingredient of a moisturizer.
[Patent Document 1] WO 9636693
[Non-Patent Document 2] Y. Hayashi, K. Yoshida, Proc. Natl. Acad. Sci. USA, Vol. 85, pp. 2618 to 2622 (1988)

### DISCLOSURE OF THE INVENTION

As described above, a commercial pectin obtained by extracting from the existing plant tissue has a molecular weight of from 6 x 10⁴ to 2 x 10⁵. An object to be solved by the invention is to find out characteristics of the pectin having a high molecular weight to provide a method whereby the pectins having a high molecular weight can be supplied in a large amount.

In order to solve the above objects, the present inventors have examined pectins produced by several plants and conducted an extensive study on a method for producing such pectins in a large amount in order to obtain pectins having a much higher molecular weight as compared with the existing pectins and, as a result, have found that pectins having a much higher molecular weight as compared with the conventional commercial pectins could be obtained and produced in a large amount by inducing callus with a Nigella plant as a raw material plant and further culturing cells. They have further found out that the obtained pectin having a much higher molecular weight could be used as a moisturizer because the capability of maintaining moisture is excellent. The present invention has been completed on the basis of such knowledge.

That is, the present invention is specified by the following matters.
[1] a pectin comprising a galacturonic acid of from 70 to 90 mole % as a constituent sugar, and wherein the molecular weight measured by the gel filtration chromatography is in the range of 1 x 10⁴ to 5 x 10⁷ and the peak molecular weight is in the range of 2 x 10⁵ to 1 x 10⁷;
[2] the pectin as described in [1], wherein the mole ratio of the constituent sugars of a galacturonic acid : arabinose : galactose : glucuronic acid : glucose : xylose : rhamnose : mannose : fructose is 7 to 9 : 0.4 to 0.7 : 0.4 to 0.6 : 0.1 to 0.6 : 0.05 to 0.5 : 0.04 to 0.4 : 0 to 0.1 : 0 to 0.08 : 0 to 0.05;
[3] the pectin as described in [1] or [2], wherein the pectin is separated and recovered from the culture or the culture broth obtained by culturing a Nigella plant cell;
[4] the pectin as described in [3], wherein the Nigella plant is Nigella damascena or Nigella sativa;
[5] use of the pectin as described in any one of [1] to [4] as a moisturizer;
[6] a cosmetic material containing the pectin as described in any one of [1] to [4]; and
[7] a process for preparing the pectin as described in any one of [1] to [4], which comprises separating and recovering the pectin as described in any one of [1] to [4] from the culture or the culture broth obtained by culturing the Nigella plant cell.

The plant cell-derived pectin of the present invention has a high molecular weight as compared with the commercial pectins obtained by the conventional methods. For that reason, the pectin of the present invention is superior in the capability of maintaining moisture. Furthermore, the gel strength is also excellent and the viscosity is low. When the pectin of the present invention is used as a cosmetic material, the pectin exhibits a good feeling to use it and is optimum as a moisturizer of a cosmetic material.

### BRIEF DESCRIPTION OF THE DRAWINGS

Fig. 1 is a view (graph) illustrating the results from Example 12(2). In the drawing, A refers to the range of the molecular weight, while B refers to the peak of the molecular weight.

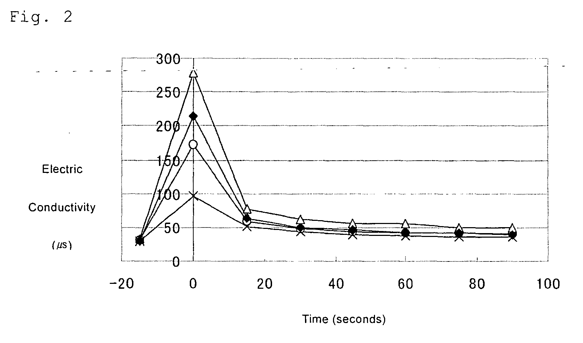

Fig. 2 is a view (graph) illustrating the results from Test Example 2. In the drawing, the triangle indicates the pectin of the present invention, the square indicates sodium hyarulonate, the circle indicates the commercial pectin, and x indicates water.

### BEST MODE FOR CARRYING OUT THE INVENTION

The pectin of the present invention is obtained by separating and recovering from the culture or the culture broth which is obtained by culturing a plant cell. The pectin is preferably obtained by separating and recovering from the culture or the culture broth which is obtained by culturing a Nigella plant cell.

The Nigella plant belongs to Ranunculaceae and there exist about 20 species in the Mediterranean, West Asia and North America as the places of origin. Typical examples of the species include Nigella damascena, Nigella sativa, Nigella hispanica and Nigella arvensis. In the present invention, any of the plants belonging to Nigella may be used, but preferably used are Nigella damascena and Nigella sativa.

The pectin of the present invention can be prepared by two-stage culture comprising inducing callus (dedifferentiated cell) and further proliferating the obtained callus.

First, in the culture for inducing callus, tissues such as a seed, a leaf, a stem, a root and the like of the Nigella plant are sterilized for their surfaces with 30% to 95% of ethanol, 0.01% to 0.1% of benzalkonium chloride, 0.1% to 5% of sodium hypochlorite or the like, and callus is inducing and cultured. A medium for inducing callus is used with a carbon source and a plant hormone added to the medium components generally used for plant tissue culture, such as Murashige - Skoog, Linsmaier - Skoog, White, Nitsch, Gamborg, WPM (Woody Plant Medium) and the like, and vapor heated under a condition of a temperature of 121°C for 15 minutes for sterilizing. Examples of the carbon source include monosaccharide such as glucose, fructose and the like, disaccharide such as sucrose, maltose and the like having such monosaccharide as constituent sugars or oligosaccharides. The amount of the carbon source is used in the range of 0.1% to 10%. Of these sugars, however, sucrose is preferably used in the range of 0.5% to 5%. As the plant hormone, auxines, cytokinin, gibberellin, abscisic acid, brassinosteroid and the like are used singly or in combination in the concentration range of 0 to 10⁻⁴ M. Examples of auxines include indoleacetic acid, indolebutyric acid, α-naphthaleneacetic acid, 2,4-dichlorophenoxy acetic acid and 2,6-dichlorobenzoic acid. Further, examples of cytokinin include zeatin, furfurylaminopurine (kinetin), benzyladenine, isopentenyladenine, dimethylaminopurine and the like. Preferable examples of gibberellin include GA₁, GA₃, GA₄ and GA₇ of activated types. A preferable example of brassinosteroid includes brassinolide. Of the ingredients added to the medium used for callus induction, substances decomposed by heating are added after further filtering using a filter of 0.2 µl for sterilization, after vapor heating the other ingredients for sterilization. pH in the medium is from 5 to 7, and preferably from 5.5 to 6 by a dilute alkaline solution such as sodium hydroxide, potassium hydroxide or the like at the time of production thereof.

A callus induction can be carried out either in a solid medium or in a liquid medium, but it is preferably cultured on a solid medium in which the above medium is solidified by 0.4 to 2% agar, 0.1 to 0.5% Gelrite or the like. Culture is carried out at a temperature of from 15°C to 30°C and preferably from 23°C to 27°C, and at that time, it may be carried out under any conditions of either a bright place or a dark place. Usually, callus can be obtained as a cell cluster having a diameter of 1 to 30 mm after 5 to 60 days from the culture.

Next, callus is transplanted into a new medium, whereby callus is proliferated by cultivation thereof. The culture may be any of a solid culture or a liquid culture, but considering the economic efficiency and mass productivity, a liquid culture is preferable. The culture may be carried out on the same medium as the medium used at the time of callus induction or on a medium modified such that a salt concentration is lowered or the like. The medium modified such that a salt concentration is lowered refers to a medium in which one, two or more ingredients of main inorganic salts such as potassium nitrate, sodium nitrate, ammonium nitrate, sodium dihydrogen phosphate, calcium chloride, magnesium sulfate and the like in the medium ingredients are reduced in the ranges in which production of pectins is not adversely affected. Further, in the medium, an organic acid such as a casamino acid or the like, a nitrogen source such as an amino acid or the like, coconut milk, yeast extract and polypepton, known as a well-known additive, can also be added in a concentration of from 0.01 to 10 g/l.

The culture temperature, light condition and culture period for proliferating callus are the same as the culture conditions for callus induction. By repeating the culture 2 to 5 times in that culture condition, a culture cell is obtained as a cell cluster that is produced by several cells to several hundreds of cells. The culture cell is subsequently further proliferated, whereby pectins can be produced in a colloidal state or in a soluble state in the culture or the culture broth. In order to produce pectins in a large amount, a liquid culture is preferable. Its culture method includes a batch culture comprising recovering the whole quantity of the culture and the culture broth every 5 to 30 days, a semi-batch culture comprising recovering a part of the culture and the culture broth every 5 to 30 days and adding a fresh medium for culturing, and a continuous culture comprising always carrying out the recovery of the culture and the culture broth, and addition of a fresh medium at a predetermined ratio. Any of these methods may be used. The culture can be carried out at an initial cell density in the range of from 1 to 300 g/l, but it is also possible to produce high-concentration pectins within a short period of time by a high density of the cell. To supply oxygen for the liquid culture, any of reciprocal shaking and gyratory shaking, direct ventilation to the culture broth or an indirect method using a hollow fiber can be used. In order to enhance the productivity of pectins, it is effective to increase the amount of oxygen to be supplied. For example, in the gyratory shake culture, the frequency of shaking is preferably not less than 100 rpm not more than 200 rpm, or the amount of a medium of about a flask vessel is preferably not less than 5% not more than 30% of the capacity for increasing the liquid-air interface, and when directly ventilating to the culture broth, the amount of ventilation of about the culture broth is suitably not less than 5 volume % not more than 20 volume % per minute. An increase in the amount of oxygen to be supplied is also effective to promote the proliferation rate of cells.

The pectin in the culture or the culture broth thus obtained exists in a colloidal state or in a soluble state.

In the culture of plant cells, a production method for culturing cells of the Nigella plants particularly brings about the production of pectins in a large amount.

In order to recover the pectin from the culture or the culture broth, the pectin in a colloidal state or in a soluble state is first solubilized. At the time of solubilization, a dilute alkaline solution such as water, a chelating agent, an acid buffer agent or carbonate is added into the culture and the culture broth such that the concentration of the pectin becomes not more than 3 g/l. The pectin is solubilized under a mild temperature condition of from 0°C to 40°C and preferably from 15°C to 35°C. Examples of the chelating agent include oxalic acid, ammonium oxalate, polyphosphate (sodium hexametaphosphate or calgon of food additives), ethylenediaminetetracetic acid, salts thereof and the like. Examples of the acid buffer agent include phosphoric acid, citric acid and the like. Examples of the carbonate include sodium carbonate, potassium carbonate and the like.

After the solubilization of the pectin, a solid content of the cell or the like is removed by filtering or centrifugation from a solution containing the pectin. Then, an organic solvent such as ethanol, acetone or the like is added in an amount of from 0.5 to 5 times its volume (V/V) and preferably from 1 to 3 times its volume (V/V) of the solution, and the pectin is precipitated and recovered. The recovered precipitate is again dissolved in water, whereby the pectin can be concentrated in an arbitrary concentration. Further, as needed, by removing the low molecular weight components using a dialysis membrane and an ultrafiltration membrane capable of appropriately fractionizing the molecular weight, the pectin having an arbitrary molecular weight is obtained. The concentrated pectin solution is freeze-dried, whereby the pectin can be obtained as a white or light yellow powder.

The pectin thus obtained is in the range of the molecular weight of from 1 x 10⁴ to 5 x 10⁷, and has the peak molecular weight of from 2 x 10⁵ to 1 x 10⁷. More preferably, the pectin is in the range of the molecular weight of from 1 x 10⁵ to 1 x 10⁷, and has the peak molecular weight of from 1 x 10⁶ to 5 x 10⁶. The molecular weight of the pectin mentioned in the present invention refers to the molecular weight measured by the gel filtration chromatography. The range of the molecular weight refers to the lower limit and upper limit of the molecular weight distribution of the pectin measured by the gel filtration chromatography. In addition, the peak molecular weight refers to the molecular weight having the highest frequency point in the molecular weight distribution of the pectin measured by the gel filtration chromatography.

The molecular weight of the pectin is determined by comparing the distribution curve of the pectin obtained as a result of the measurement by the gel filtration chromatography with the distribution curve of a standard substance having an already known molecular weight obtained as a result of the measurement by the gel filtration chromatography. The gel filtration chromatography for measuring the molecular weight of the pectin mentioned in the present invention is measured according to the following conditions.

The measurement conditions of the gel filtration chromatography for obtaining the molecular weight of the pectin of the present invention are as follows. Incidentally, in the following conditions, a differential refractive index detector is a detector to detect a substance by the difference in the refractive indexes with a mobile phase, while RID-10A manufactured by Shimadzu Corporation or its equivalent is used herein.

| | |
|---|---|
| Column: | TSKgel PW series manufactured by Tosoh Corporation or its equivalent (7.8 mm (internal diameter) x 300 mm (length)) |
| Filler: | Hydrophilic vinyl polymer gel |
| Mobile phase: | 0.1M sodium nitrate aqueous solution |
| Flow rate: | 0.5 ml/min |
| Temperature: | 40°C |
| Sample concentration: | Aqueous solution of a concentration of 1 g/l |
| Sample input amount: | 10 µl |
| Detector: | Differential refractive index detector |

Under the aforementioned conditions, a sample containing the pectin and a standard substance is measured by the gel filtration chromatography. A calibration curve is prepared using the results from the measurement of the standard substance. The molecular weight of the pectin in the sample is obtained as a relative value in terms of the standard substance by applying the results from the measurement of the sample to the calibration curve.

A method for obtaining the molecular weight from the measured values is specifically described below.

Examples of the standard substance used include polyethylene glycol (molecular weight: 2 x 10⁶, a reagent manufactured by Wako Pure Chemical Industries, Ltd.), polyethylene oxide (molecular weight: 1 x 10⁶, 5 x 10⁵, 2 x 10⁵, 5 x 10⁴, a reagent manufactured by GL Science Co., Ltd.) and pullulan (molecular weight: 1.6 x 10⁶, 8 x 10⁵, 4 x 10⁵, 2 x 10⁵, a reagent manufactured by Showa Denko K.K.). Among the above standard substances, at least 3 kinds of the standard substances having different molecular weights are properly selected and used accordingly.

When the pectin is measured by the gel filtration chromatography under the above conditions, a distribution curve peculiar to the pectin relative to an axis of the hold time is obtained. Further, when a standard substance having a known molecular weight is measured by the gel filtration chromatography under the same conditions, a distribution curve peculiar to the standard substance relative to an axis of the hold time is obtained. Incidentally, in this case, the hold time means the same as the elution amount of the mobile phase (eluant). In the distribution curve of the sample or the standard substance, the hold time of a position indicating the peak is specified as a hold time native to each substance.

The calibration curve is prepared from a relationship between the hold time peculiar to the standard substance and the molecular weight of the standard substance, and the hold time peculiar to the pectin of the sample is applied to the calibration curve for determining the molecular weight of the pectin. When the pectin having a molecular weight of not less than 2 x 10⁶ not more than 5 x 10⁴ is measured, the molecular weight of the pectin is determined by extrapolating and interpolating the above calibration curve.

In the constituent sugars of the pectin, an uronic acid in which a hydroxymethyl group at the end of the carbon chain is oxidized to a carboxyl group can be subjected to colorimetric quantification (absorbed at 530 nm) according to the known carbazole-sulfuric acid method.

The pectin of the present invention contains an uronic acid of from 70 to 96 mole %. The composition of the constituent sugars in the pectin obtained according to the present invention can be examined by quantitatively analyzing neutral sugar and uronic acid by the high performance liquid chromatography, which are isolated after acid hydrolysis under a condition of from 50°C to 100°C for 30 minutes to 6 hours by means of hydrochloric acid, sulfuric acid, acetic acid or trifluoroacetic acid. The pectin of the present invention is composed of a galacturonic acid, arabinose, galactose, a glucuronic acid, glucose, xylose, rhamnose, mannose, fructose or the like, while the mole ratio of the main ingredients is the galacturonic acid : arabinose : galactose : glucuronic acid : glucose : xylose : rhamnose : mannose : fructose of 7 to 9 : 0.4 to 0.7 : 0.4 to 0.6 : 0.1 to 0.6 : 0.05 to 0.5 : 0.04 to 0.4 : 0 to 0.1 : 0 to 0.08 : 0 to 0.05. That is, the pectin of the present invention comprises a galacturonic acid of from 70 to 90 mole % as a main constituent sugar.

The degree of esterification of the pectin can be examined according to a method for quantitatively analyzing the saponified carboxyl group. The degree of esterification of the pectin of the present invention is from 0 to 60%.

The pectin of the present invention is a pectin having a much higher molecular weight as compared with the commercial pectin which is conventionally obtained. A new characteristic of the pectin of the present invention is a capability of maintaining high moisture as compared with the existing commercial pectin. Further, the pectin has a property of the conventional commercial pectin, i.e., solubility and a property of gelating by the contact with a divalent or higher metal salt such as calcium ion or a sugar and an acid.

The pectin of the present invention has characteristics such as a high molecular weight, a high capability of maintaining moisture as compared with the existing commercial pectin. For that reason, the pectin can be used as a cosmetic material as an effective ingredient of a moisturizer. The moisturizer mentioned herein refers to a substance to be mainly combined in a cosmetic material for preventing evaporation of moisture from the skin, for controlling moisture on the skin surface and for granting a moist feeling. In a test on maintenance of moisture according to a known paper disc method or a test on water holding capacity for corneous stratum using a skin surface hygrometer, the pectin of the present invention exhibits a capability of maintaining higher moisture than sodium hyaluronate generally used as a moisturizer in any of the tests. Since the pectin of the present invention exhibits the excellent capability of maintaining initial moisture, it is excellent in skin penetrating property or affinity to the skin. Further, even though the pectin of the present invention has a high molecular weight which is about the same as sodium hyaluronate, its viscosity is lower than sodium hyaluronate. For that reason, it gives a moist feeling or smooth feeling to users, and a less sticky skin feeling to use it. The pectin of the present invention can provide various kinds of cosmetic materials in the various forms of a solution form such as a face lotion or the like; a gel form such as a cleansing foam or the like; an emulsification form such as a milky lotion or the like; a solid form such as a soap and rouge; a powder form such as a pressed powder; a paste form such as a foundation; a film form such as a pack; an aerosol form such as a hair spray as a cosmetic material excellent in a moist feeling or smooth feeling.

In addition to the pectin of the present invention, other ingredients usually used for cosmetics, medical supplies or the like can be properly blended as necessary in the cosmetic material of the present invention; examples of such ingredients include powder ingredients, liquid fats and oils, solid fats and oils, waxes, hydrocarbons, higher fatty acids, ester oils, silicone oils, anionic surfactants, cationic surfactants, ampholytic surfactants, nonionic surfactants, water-soluble polymers, thickening agents, ultraviolet light absorbents, sequestering agents, alcohols, sugars, amino acids, organic amines, polymer emulsions, pH adjusting agents, vitamins, antioxidants, antioxidation auxiliary agents, and water; and the cosmetic material of the present invention can be prepared depending on the intended types such as a solution form cosmetic, a gel form cosmetic, an emulsification form cosmetic, a solid form cosmetic, a powder form cosmetic, a paste form cosmetic, a film form cosmetic, an aerosol form cosmetic and the like according to generally known methods. Concrete examples of the ingredient which can be blended in are mentioned below, but the cosmetic material of the present invention can be prepared by blending the pectin of the present invention and any one, two or more kinds of the following ingredients.

Examples of the powder ingredients include inorganic powders (for example, talc, kaolin, mica, sericite, muscovite, phlogopite, synthetic mica, lepidolite, biotite, vermiculite, magnesium carbonate, calcium carbonate, aluminum silicate, barium silicate, calcium silicate, magnesium silicate, strontium silicate, tungstic acid metal salt, magnesium, silica, zeolite, barium sulfate, firing calcium sulfate (calcined gypsum), calcium phosphate, fluorine apatite, hydroxy apatite, ceramic powder, metallic soaps (for example, myristic acid zinc, calcium palmitate, and aluminum stearate), boron nitride and the like); organic powders (for example, polyamide resin powder (nylon powder), polyethylene powder, poly methyl methacrylate powder, polystyrene powder, powders of copolymer resin of styrene and acrylic acid, benzoguanamine resin powder, polytetrafluoroethylene powder, cellulose powder and the like); inorganic white pigments (for example, titanium dioxide, zinc oxide and the like); inorganic red pigments (for example, iron oxide (red iron oxide; Bengala), iron titanate and the like); inorganic brown pigments (for example, γ-iron oxide and the like); inorganic yellow pigments (for example, yellow iron oxide, loess and the like); inorganic black pigments (for example, black iron oxide, low oxides of titanium and the like); inorganic purple pigments (for example, manganese violet, cobalt violet and the like); inorganic green pigments (for example, chromium oxide, chromium hydroxide, cobalt titanate and the like); inorganic blue pigments (for example, ultramarine blue, iron blue and the like); pearl pigments (for example, titanium oxide-coated mica, titanium oxide-coated bismuth oxychloride, titanium oxide-coated talc, colored titanium oxide-coated mica, bismuth oxychloride, fish scale foils and the like); metal powder pigments (for example, aluminium powder, copper powder and the like); organic pigments such as zirconium, barium, aluminium rake or the like (for example, organic pigments such as red 201, red 202, red 204, red 205, red 220, red 226, red 228, red 405, orange 203, orange 204, yellow 205, yellow 401 and blue 404, as well as red 3, red 104, red 106, red 227, red 230, red 401, red 505, orange 205, yellow 4, yellow 5, yellow 202, yellow 203, green 3, blue 1 and the like); and natural colors (for example, chlorophyll, β-carotene and the like).

Examples of the liquid fats and oils include avocado oil, tsubaki oil, turtle oil, macadamia nut oil, corn oil, mink oil, olive oil, rape seed oil, egg yolk oil, sesame oil, persic oil, wheat germ oil, sasanqua oil, castor oil, linseed oil, safflower oil, cotton seed oil, perilla oil, soybean oil, peanut oil, tea seed oil, Japanese nutmeg oil (kaya oil), rice bran oil, Chinese gimlet oil, Japan gimlet oil, jojoba oil, germ oil, triglycerin and the like.

Examples of the solid fats and oils-include cacao butter, coconut oil, hydrogenated coconut oil, palm oil, palm kernel oil, Japanese core wax nucleus oil, hydrogenated oil, Japanese core wax, hydrogenated castor oil and the like.

Examples of the waxes include yellow beeswax, candelilla wax, cotton wax, carnauba wax, bayberry wax, ibota wax, whale wax, montan wax, bran wax, lanolin, kapok wax, lanolin acetate, liquid lanolin, sugar cane wax, lanolin fatty acid isopropyl, hexyl laurate, reduced lanolin, jojoba wax, hard lanolin, shellac wax, POE lanolin alcohol ether, POE lanolin alcohol acetate, POE cholesterol ether, lanolin fatty acid polyethylene glycol, POE hydrogenated lanolin alcohol ether and the like.

Examples of the hydrocarbon oils include liquid paraffin, ozocerite, squalane, pristane, paraffin, ceresin, squalene, vaseline, microcrystalline wax and the like.

Examples of the higher fatty acids include lauric acid, myristic acid, palmitic acid, stearic acid, behenic acid, oleic acid, undecylenic acid, tall oil fatty acid, isostearic acid, linolic acid, linoleic acid, eicosapentaenoic acid (EPA), docosahexaenoic acid (DHA) and the like.

Examples of the ester oils include isopropyl myristate, cetyl octanoate, octyl dodecyl myristate, isopropyl palmitate, butyl stearate, hexyl laurate, myristil myristate, decyl oleate, dimethyl hexyl decyl octanoate, cetyl lactate, myristil lactate, lanolin acetate, iso cetyl stearate, iso cetyl isostearate, cholesteryl hydroxy 12-stearate, di-2-ethylene glycol ethylhexanoate, dipentaerythritol fatty acid ester, n-alkyl glycol monoisostearate, neopentyl glycol dicaprate, diisostearyl malate, glycerin di-2-heptylundecanoate, trimethylolpropane tri-2-ethylhexanoate, trimethylolpropane triisostearate, tetra-2-pentaerythritol ethylhexanoate, glycerin tri-2-ethylhexanoate, glycerin trioctanoate, glycerin triisopalmitate, trimethylolpropane triisostearate, cetyl 2-ethyl hexanoate, 2-ethylhexyl palmitate, glycerin trimyristate, tri-2-heptyl undecanoate glyceride, methyl castor oil fatty acid ester, oleyl oleate, aceto glyceride, 2-heptyl undecyl palmitate, diisobutyl adipate, 2-octyldodecyl N-lauroyl-L-glutamate, di-2-heptyl undecyl adipate, ethyl laurate, di-2-ethylhexyl sebacate, 2-hexyl decyl myristate, 2-hexyl decyl palmitate, 2-hexyl decyl adipate, diisopropyl sebacate, 2-ethylhexyl succinate, triethyl citrate and the like.

Examples of the silicone oils include chained polysiloxanes (for example, dimethyl polysiloxane, methylphenyl polysiloxane, diphenyl polysiloxane and the like); cyclic polysiloxanes (for example, octamethyl cyclotetra siloxane, decamethyl cyclopenta siloxane, dodecamethyl cyclohexa siloxane and the like), silicone resins forming a three-dimensional network structure, silicone rubbers, and various modified polysiloxanes (amino-modified polysiloxane, polyether-modified polysiloxane, alkyl-modified polysiloxane, fluorine-modified polysiloxane and the like).

Examples of the anionic surfactants include fatty acid soaps (for example, sodium laurate, sodium palmitate and the like); higher alkyl sulfate ester salts (for example, sodium lauryl sulfate, potassium lauryl sulfate and the like); alkylether sulfate ester salts (for example, POE-triethanolamine lauryl sulfate, sodium POE-lauryl sulfate and the like); N-acyl sarcosinic acids (for example, sodium lauroyl sarcosinate and the like); higher fatty acid amide sulfonates (for example, sodium N-myristoyl-N-methyl taurate, sodium methyl cocoyl taurate, sodium lauryl methyl taurate and the like); phosphate salts (sodium POE-oleyl ether phosphate, POE-stearyl ether phosphoric acid and the like); sulfosuccinates (for example, sodium di-2-ethylhexyl sulfosuccinate, sodium monolauroyl monoethanol amide polyoxyethylene sulfosuccinate, sodium lauryl polypropylene glycol sulfosuccinate and the like); alkylbenzene sulfonates (for example, sodium linear dodecylbenzenesulfonate, triethanolamine linear dodecylbenzenesulfonate, linear dodecylbenzenesulfonic acid and the like); higher fatty acid ester sulfates (for example, hydrogenated palm oil fatty acid glycerine sodium sulfate and the like): N-acyl glutamates (for example, monosodium N-lauroylglutamate, disodium N-stearoylglutamate, monosodium N-myristoyl-L-glutamate and the like); sulfated oils (for example, turkey red oil and the like); POE-alkylether carboxylic acid; POE-alkylarylether carboxylate; α-olefin sulfonate; higher fatty acid ester sulfonates; sec-alcohol sulfates; higher fatty acid alkylolamide sulfates; sodium lauroyl monoethanolamide succinates; ditriethanolamine N-palmitoylaspartate; sodium caseinate and the like.

Examples of the cationic surfactants include alkyltrimethyl ammonium salts (for example, stearyltrimethyl ammonium chloride, lauryltrimethyl ammonium chloride and the like); alkylpyridinium salts (for example, cetylpyridinium chloride and the like); distearyldimethylammonium dialkyldimethylammonium chloride; poly (N,N'-dimethyl-3,5-methylene piperidinium) chloride; alkyl quaternary ammonium salts; alkyl dimethylbenzyl ammonium salts; alkyl isoquinolinium salts; dialkylmorphonium salts; POE-alkylamines; alkylamine salts; polyamine fatty acid derivatives; amylalcohol fatty acid derivatives; benzalkonium chloride; benzethonium chloride and the like.

Examples of the ampholytic surfactants include imidazoline type ampholytic surfactants (for example, 2-undecyl-N,N,N-(hydroxyethyl carboxymethyl)-2-imidazoline sodium salt, 2-cocoyl-2-imidazolinium hydroxide-1-carboxyethyloxy 2 sodium salt and the like); and betaine type surfactants (for example, 2-heptadecyl-N-carboxymethyl-N-hydroxyethyl imidazolinium betaine, lauryldimethylamino betaine acetate, alkyl betaine, amide betaine, sulfobetaine and the like).

Examples of the lipophilic nonionic surfactant of nonionic surfactants include sorbitan fatty acid esters (for example, sorbitan monooleate, sorbitan monoisostearate, sorbitan monolaurate, sorbitan monopalmitate, sorbitan monostearate, sorbitan sesquioleate, sorbitan trioleate, diglycerol sorbitan penta-2-ethylhexylate, diglycerol sorbitan tetra-2-ethylhexylate and the like); glycerin polyglycerin fatty acids (for example, mono cottonseed oil fatty acid glycerin, glycerin monoerucate, glycerin sesquioleate, glycerin monostearate, α,α'-glycerin oleate pyroglutamate, monostearate glycerin malic acid and the like); propylene glycol fatty acid esters (for example, propylene glycol monostearate and the like); hydrogenated castor oil derivatives; glycerin alkylethers and the like.

Examples of the hydrophilic nonionic surfactant of nonionic surfactants include POE-sorbitan fatty acid esters (for example, POE-sorbitan monooleate, POE-sorbitan monostearate, POE-sorbitan monoolate, POE-sorbitan tetraoleate and the like); POE sorbitol fatty acid esters (for example, POE sorbitol monolaurate, POE-sorbitol monooleate, POE-sorbitol pentaoleate, POE-sorbitol monostearate and the like); POE-glycerin fatty acid esters (for example, POE-monooleate such as POE-glycerin monostearate, POE-glycerin monoisostearate, POE-glycerin triisostearate and the like); POE-fatty acid esters (for example, POE-distearate, POE-monodioleate, ethylene glycol distearate and the like); POE-alkylethers (for example, POE-lauryl ether, POE-oleyl ether, POE-stearyl ether, POE-behenyl ether, POE-2-octyl dodecyl ether, POE-cholestanol ether and the like); pluaronic types (for example, pluaronic and the like); POE/POP-alkylethers (for example, POE/POP-cetyl ether, POE/POP-2-decyl tetradecyl ether, POE/POP-monobutyl ether, POE/POP-hydrogenated lanolin, POE/POP-glycerin ether and the like); tetra POE/tetra POP-ethylenediamine condensates (for example, tetronic and the like); POE-castor oil or hydrogenated castor oil derivatives (for example, POE-castor oil, POE-hydrogenated castor oil, POE-hydrogenated castor oil monoisostearate, POE-hydrogenated castor oil triisostearate, POE-hydrogenated castor oil monopyroglutamic monoisostearic diester, POE-hydrogenated castor oil maleic acid and the like); POE-beeswax/lanolin derivatives (for example, POE-sorbitol beeswax and the like); alkanol amides (for example, coconut oil fatty acid diethanol amide, laurate monoethanolamide, fatty acid isopropanol amide and the like); POE-propylene glycol fatty acid esters; POE-alkylamines; POE-fatty acid amides; sucrose fatty acid esters; alkyl ethoxy dimethylamine oxides; trioleyl phosphoric acid and the like.

Examples of the natural water-soluble polymer of water-soluble polymers include plant type polymers (for example, gum arabic, gum tragacanth, galactan, guar gum, carob gum, karaya gum, carrageenan, agar, quince seed (Cydonia oblonga), algae colloids (brown algae extract), starches (rice, corn, potato, and wheat), and glycyrrhizic acid); microorganism type polymers (for example, xanthan gum, dextran, succinoglucan, pullulan and the like); and animal type polymers (for example, collagen, casein, albumin, gelatin and the like).

Examples of the semi-synthetic water-soluble polymer of water-soluble polymers include starch type polymers (for example, carboxymethyl starch, methylhydroxypropyl starch and the like); cellulosic polymers (for example, methyl cellulose, ethyl cellulose, methylhydroxypropyl cellulose, hydroxyethyl cellulose, cellulose sodium sulfate, hydroxypropyl cellulose, carboxymethyl cellulose, sodium carboxymethyl cellulose, crystal cellulose, cellulose powder and the like); and alginic acid type polymers (for example, sodium alginate, propylene glycol alginate and the like).

Examples of the synthetic water-soluble polymer of water-soluble polymers include vinyl type polymers (for example, polyvinyl alcohol, polyvinyl methyl ether, polyvinyl pyrrolidone, carboxy vinyl polymer and the like); polyoxyethylene type polymers (for example, a copolymer of polyethylene glycol 20,000, 40,000, or 60,000 and polyoxyethylene polyoxypropylene and the like); acrylic type polymers (for example, sodium polyacrylate, polyethyl acrylate, polyacrylamide and the like); polyethylene imine; cationic polymers and the like.

Examples of the thickening agents include gum arabic, carrageenan, karaya gum, gum tragacanth, carob gum, quince seed (Cydonia oblonga), casein, dextrin, gelatin, sodium pectate, sodium alginate, methyl cellulose, ethyl cellulose, CMC, hydroxyethyl cellulose, hydroxypropyl cellulose, PVA, PVM, PVP, sodium polyacrylate, carboxy vinyl polymer, locust bean gum, guar gum, tamarind gum, cellulose dialkyl dimethyl ammonium sulfate, xanthan gum, aluminum magnesium silicate, bentonite, hectorite, AlMg silicate (beagum), laponite, silicic acid anhydride and the like.

As the ultraviolet light absorbent, the following compounds can be mentioned.
(1) Benzoic acid type ultraviolet light absorbents
   for example, para-aminobenzoic acid (hereinafter referred to as PABA), PABA monoglycerin ester, N,N-dipropoxy PABA ethyl ester, N,N-diethoxy PABA ethyl ester, N,N-dimethyl PABA ethyl ester, N,N-dimethyl PABA butyl ester, N,N-dimethyl PABA ethyl ester and the like;
(2) Anthranilic acid type ultraviolet light absorbents
   for example, homomentyl-N-acethyl anthranilate and the like;
(3) Salicylic acid type ultraviolet light absorbents
   for example, amyl salicylate, mentyl salicylate, homomentyl salicylate, octyl salicylate, phenyl salicylate, benzyl salicylate, p-isopropanolphenyl salicylate and the like;
(4) Cinnamic acid type ultraviolet light absorbents
   for example, octyl cinnamate, ethyl-4-isopropyl cinnamate, methyl-2,5-diisopropyl cinnamate, ethyl-2,4-diisopropyl cinnamate, methyl-2,4-diisopropyl cinnamate, propyl-p-methoxy cinnamate, isopropyl-p-methoxy cinnamate, isoamyl-p-methoxy cinnamate, octyl-p-methoxy cinnamate (2-ethylhexyl-p-methoxy cinnamate), 2-ethoxyethyl-p-methoxy cinnamate, cyclohexyl-p-methoxy cinnamate, ethyl-α-cyano-β-phenyl cinnamate, 2-ethylhexyl-α-cyano-β-phenyl cinnamate, glyceryl mono-2-ethylhexanoyl-diparamethoxy cinnamaten and the like;
(5) Triazine type ultraviolet light absorbents
   for example, bisresorcinyltriazine; more specifically, bis{[4-2-ethylhexyloxy]-2-hydroxy]phenyl}-6-(4-methoxyphenyl)-1,3,5-triazine, 2,4,6-tris{4-(2-ethylhexyloxycarbonyl)anilino}-1,3,5-triazine and the like; and
(6) Other ultraviolet light absorbents
   for example, 3-(4'-methylbenzylidene)-d,1-camphor, 3-benzylidene-d,1-camphor, 2-phenyl-5-methyl benzoxazole, 2,2'-hydroxy-5-methylphenyl benzotriazole, 2-(2'-hydroxy-5'-t-octylphenyl) benzotriazole, 2-(2'-hydroxy-5'-methylphenyl benzotriazole, dibenzaladine, dianisoylmethane, 4-methoxy-4'-t-butyldibenzoylmethane, 5-(3,3-dimethyl-2-norbornylidene)-3-pentane-2-one and the like; pyridazine derivatives such as dimorpholino pyridazinone and the like.

Examples of the sequestering agents include 1-hydroxy ethane-1,1-diphosphonic acid, 1-hydroxy ethane-1,1-diphosphonic acid tetrasodium salt, disodium edetate, trisodium edetate, tetrasodium edetate, sodium citrate, sodium polyphosphate, sodium metaphosphate, gluconic acid, phosphoric acid, citric acid, ascorbic acid, succinic acid, edetic acid, trisodium ethylenediaminehydroxyethyl triacetate and the like.

Examples of the lower alcohols of alcohols include ethanol, propanol, isopropanol, isobutyl alcohol, t-butyl alcohol and the like.

Examples of the polyhydric alcohols of alcohols include dihydric alcohols (for example, ethylene glycol, propylene glycol, trimethylene glycol, 1,2-butylene glycol, 1,3-butylene glycol, tetramethylene glycol, 2,3-butylene glycol, pentamethylene glycol, 2-butene-1,4-diol, hexylene glycol, octylene glycol and the like); trihydric alcohols (for example, glycerin, trimethylolpropane and the like); tetrahydric alcohols (for example, pentaerythritol including 1,2,6-hexanetriol and the like); pentahydric alcohols (for example, xylitol and the like); hexahydric alcohols (for example, sorbitol, mannitol and the like); polyhydric alcohol polymers (for example, diethylene glycol, dipropylene glycol, triethylene glycol, polypropylene glycol, tetraethylene glycol, diglycerin, polyethylene glycol, triglycerin, tetraglycerin, polyglycerin and the like); dihydric alcohol alkyl ethers (for example, ethylene glycol monomethyl ether, ethylene glycol monoethyl ether, ethylene glycol monobutyl ether, ethylene glycol monophenyl ether, ethylene glycol monohexyl ether, ethylene glycol mono2-methylhexyl ether, ethylene glycol isoamyl ether, ethylene glycol benzyl ether, ethylene glycol isopropyl ether, ethylene glycol dimethyl ether, ethylene glycol diethyl ether, ethylene glycol dibutyl ether and the like); dihydric alcohol alkyl ethers (for example, diethylene glycol monomethyl ether, diethylene glycol monoethyl ether, diethylene glycol monobutyl ether, diethylene glycol dimethyl ether, diethylene glycol diethyl ether, diethylene glycol butyl ether, diethylene glycol methylethyl ether, triethylene glycol monomethyl ether, triethylene glycol monoethyl ether, propylene glycol monomethyl ether, propylene glycol monoethyl ether, propylene glycol monobutyl ether, propylene glycol isopropyl ether, dipropylene glycol methyl ether, dipropylene glycol ethyl ether, dipropylene glycol butyl ether and the like); dihydric alcohol ether esters (for example, ethylene glycol monomethyl ether acetate, ethylene glycol monoethyl ether acetate, ethylene glycol monobutyl ether acetate, ethylene glycol monophenyl ether acetate, ethylene glycol diadipate, ethylene glycol disuccinate, diethylene glycol monoethyl ether acetate, diethylene glycol monobutyl ether acetate, propylene glycol monomethyl ether acetate, propylene glycol monoethyl ether acetate, propylene glycol monopropyl ether acetate, propylene glycol monophenyl ether acetate and the like); glycerin mono alkyl ethers (for example, chimyl alcohol, selachyl alcohol, batyl alcohol and the like); sugar alcohols (for example, sorbitol, maltitol, maltotriose, mannitol, sucrose, erythritol, glucose, fructose, starch amylolysis sugar, maltose, xylitose, alcohol prepared by the reduction of starch amylolysis sugar and the like); glysolid; tetrahydrofurfuryl alcohol; POE-tetrahydrofurfuryl alcohol; POP-butyl ether; POP/POE-butyl ether; Tripoli oxypropylene glycerin ether; POP-glycerin ether; POP-glycerin ether phosphoric acid; POP/POE-pentane erythritol ether, polyglycerin and the like.

Examples of the higher alcohols include straight chain alcohols (for example, lauryl alcohol, cetyl alcohol, stearyl alcohol, behenyl alcohol, myristyl alcohol, oleyl alcohol, cetostearyl alcohol and the like); and branched chain alcohols (for example, mono stearyl glycerin ether (batyl alcohol), 2-decyltetradecinol, lanolin alcohol, cholesterol, phytosterol, hexyldodecanol, isostearyl alcohol, octyldodecanol and the like).

Examples of the monosaccharides of sugars include trioses (for example, D-glyceryl aldehyde, dihydroxyacetone and the like); tetroses (for example, D-erythrose, D-erythrulose, D-threose, erythritol and the like); pentoses (for example, L-arabinose, D-xylose, L-lyxose, D-arabinose, D-ribose, D-ribulose, D-xylulose, L-xylulose and the like); hexoses (for example, D-glucose, D-talose, D-psicose, D-galactose, D-fructose, L-galactose, L-mannose, D-tagatose and the like); heptoses (for example, aldoheptose, heprose and the like); octoses (for example, octurose and the like); deoxysugar (for example, 2-deoxy-D-ribose, 6-deoxy-L-galactose, 6-deoxy-L-mannose and the like); amino sugar (for example, D-glucosamine, D-galactosamine, sialic acid, amino uronic acid, muramic acid and the like); and uronic acid (for example, D-glucuronic acid, D-mannuronic acid, L-guluronic acid, D-galacturonic acid, L-iduronic acid and the like).

Examples of the oligosaccharides of sugars include sucrose, umbelliferose, lactose, planteose, isolignoses, α,α-trehalose, raffinose, lignoses, umbilicine, stachyose, verbascose and the like.

Examples of the polysaccharides of sugars include cellulose, quince seed, chondroitin sulfuric acid, starch, galactan, dermatan sulfuric acid, glycogen, gum arabic, heparan sulfuric acid, hyaluronic acid, traganth gum, keratan sulfuric acid, chondroitin, xanthan gum, mucoitin sulfuric acid, guar gum, dextran, keratan sulfuric acid, locust bean gum, succinoglucan, charonic acid and the like.

Examples of the amino acids include neutral amino acids (for example, threonine, cysteine and the like) and basic amino acids (for example, hydroxylysine and the like). Meanwhile, examples of the amino acid derivatives include sodium acyl sarcosinate (sodium lauroyl sarcosinate), acyl glutamate, acyl β-sodium alanine, glutathione, pyrrolidone carboxylic acid and the like.

Examples of the organic amines include monoethanolamine, diethanolamine, triethanolamine, morpholine, triisopropanolamine, 2-amino-2-methyl-1,3-propanediol, 2-amino-2-methyl-1-propanol and the like.

Examples of the polymer emulsions include acrylic resin emulsion, ethyl polyacrylate emulsion, acryl resin liquid, polyacrylic alkyl ester emulsion, polyvinyl acetate resin emulsion, natural rubber latex and the like.

Examples of the pH adjusting agents include buffers such as lactic acid-sodium lactate, citric acid-sodium citrate, succinic acid-sodium succinate and the like.

Examples of the vitamins include vitamin A, B1, B2, B6, C and E as well as their derivatives, pantothenic acid and its derivatives, biotin and the like.

Examples of the antioxidants include tocopherols, dibutyl hydroxytoluene, butyl hydroxyanisole, gallic esters and the like.

Examples of the antioxidation auxiliary agents include phosphoric acid, citric acid, ascorbic acid, maleic acid, malonic acid, succinic acid, fumaric acid, cephalin, hexametaphosphate, phytic acid, ethylene diamine tetraacetic acid and the like.

The pectin of the present invention and other moisturizers may be used together for the cosmetic material of the present invention. Examples of such moisturizers include polyethylene glycol, propylene glycol, glycerin, 1,3-butylene glycol, xylitol, sorbitol, maltitol, chondroitin sulfuric acid, hyaluronic acid, mucoitin sulfuric acid, caronic acid, atelocollagenous protein, cholesteryl-12-hydroxystearate, sodium lactate, bile salt, d1-pyrrolidone carboxylates, short chain soluble collagenous protein, diglycerin (EO) PO adducts, chestnut rose (Rosa roxburghii) extract, Yarrow (Achillea millefolium) extract, Yellow Sweet clover (Melilotus officinalis) extract and the like.

Examples of other possible ingredients include antiseptics (methylparaben, ethylparaben, butylparaben, phenoxyethanol and the like); anti-inflammatory agents (for example, glycyrrhizic acid derivatives, glycyrrhetinic acid derivatives, salicylic acid derivatives, hinokitiol, zinc oxide, allantoin and the like); whitening agents (for example, saxifraga stolonifera extract, arbutin and the like); various extracts (for example, phellodendron bark, coptis rhizome, lithospermum root, Paeonia lactiflora, Swertia japonica, Birch, sage, Japanese loquat, carrot, aloe, Malva sylvestris, Iris, grape, coix seed, sponge gourd, lily, saffron, Cnidium officinale, ginger, Hypericum erectum, Ononis, garlic, Guinea pepper, citrus unshiu peel, Angelica acutiloba, seaweed and the like); activators (for example, royal jelly, photosensitive substances, cholesterol derivatives and the like); blood circulation promoting agents (for example, nicotinic acid benzyl esters, nicotinic acid β-butoxy ethyl esters, capsaicin, zingerone, cantharis tincture, ichthammol, tannic acid, α-borneol, tocopherol nicotinate, inositol hexanicotinate, cyclandelate, cinnarizine, tolazoline, acetylcholine, verapamil, cepharanthine, γ-orizanol and the like); anti-seborrhea agents (for example, sulfur, thiantol and the like); anti-inflammatory agents (for example, tranexamic acid, thiotaurine, hypotaurine and the like); and galenicals (for example, carqueja (South America), palo azul (South America), macelamista (South America)) and the like. It is also possible to provide multi-functional cosmetic materials by combining the pectin of the present invention with one or more ingredients as described above.

The pectin of the present invention is blended in the amount of from 0.0001 to 50 weight %, preferably from 0.001 to 20 weight %, more preferably from 0.01 to 10 weight %, and particularly preferably from 0.02 to 10 weight %, based on the total amount of the cosmetic materials. When the blending amount is less than 0.0001 weight % based on the cosmetic materials, moisture capability becomes insufficient. When the amount exceeding 50 weight % is blended, it is not possible to expect the increased effect.

The cosmetic material thus obtained is excellent in the moisture capability, and provides a moist feeling or smooth feeling to users and a less sticky skin feeling to use it.

Furthermore, the pectin of the present invention is used as the above moisturizer. In addition thereto, it can be used the same as the conventional commercial pectin in the fields of foods such as a sticky substance of jam or marmalade, a stabilizer of beverage, a gelling agent of pudding or cream, and also in the fields of medical supplies.

### EXAMPLES

The present invention is now more specifically illustrated below with reference to Examples and the like. However, the present invention is not limited to these

### Examples.

### Example 1

A seedling of Nigella damascena was surface-sterilized by 70% ethanol and subsequently treated with sodium hypochlorite (1% of effective chlorine concentration) for 15 minutes, and then washed with distilled water three times. Distilled water was prepared by vapor autoclaving at 121°C for 15 minutes. The surface-sterilized seedling of Nigella damascena was axenically bedded on a solid medium obtained by solidifying a WPM medium (pH adjusted to 5.7 by 0.1M sodium hydroxide) shown in Table 1 containing 3% sucrose and 10⁻⁵M α-naphthyl acetate as a plant hormone with 0.8% agar. An amorphous dedifferentiated cell (callus) was induced after cultured at a dark place at 25°C for 1 week. A part of the obtained callus was separated and bedded on the solid medium of the same composition as that used for deriving the callus for proliferation. This step was repeated three times and a stably proliferating cell line was obtained.

**[Table 1]**

| Ingredients | Weight concentration mg/l |
|---|---|
| sodium nitrate | 1500 |
| potassium sulfate | 495 |
| calcium chloride / 2H₂O | 96 |
| calcium nitrate | 556 |
| magnesium sulfate / 7H₂O | 370 |
| potassium dihydrogen phosphate | 170 |
| sodium iron EDTA | 42 |
| boric acid | 6.2 |
| manganese sulfate / 4H₂O | 22.3 |
| zinc sulfate / 7H₂O | 8.6 |
| molybdic acid Na / 2H₂O | 0.25 |
| copper sulfate / 5H₂O | 0.25 |
| inositol | 100 |
| nicotinic acid | 0.5 |
| L-glycine | 2 |
| pyridoxine chloride | 0.5 |
| thiamine chloride | 1 |
| sodium α-naphthalene acetate | 2.08 |
| sucrose | 30000 |

### Example 2

The cell induced in Example 1 was suspended and cultured in a liquid medium of the composition in Table 1. The culture conditions were the medium amount of 300 ml applied in a 3-L flask, 25°C, a dark place, a cell density of 9.6 g/l, a gyratory shake culture of 100 rpm, and a culture period of 3 weeks. A chelating agent CyDTA (trans-1,2-diaminocyclohexane-N,N,N',N'-tetraacetic acid hydrate) was added to the culture broth such that it became 25 mM, analyzed by the high performance liquid chromatography (column in use: TSKgelG5000PWXL manufactured by Tosoh Corporation) to obtain a pectin concentration of 2.07 g/l and a cell growth rate of 42.8 times.

### Examples 3 and 4

The culture was carried out in the same manner as in Example 2 with a medium amount of 300 ml set to 500 ml and 800 ml to the 3-L flask to obtain pectins respectively. The results were shown in Example 2.

**[Table 2]**

| Example Nos. | 2 | 3 | 4 |
|---|---|---|---|
| Medium amount (ml) | 300 | 500 | 800 |
| Pectin concentration (g/l) | 2.07 | 1.58 | 1.08 |
| Growth rate (times) | 42.8 | 29.0 | 28.0 |

### Example 5

The cell induced in Example 1 was suspended in a liquid medium of the composition in Table 1 and cultured under the condition of high density of the pectin density. The culture conditions were the medium amount of 40 ml applied in a 300-mL flask, 25°C, a dark place, a cell density of 200 g/l, a gyratory shake culture of 1100 rpm, and a culture period of 1 week. The culture broth was analyzed in the same manner as in Example 2 to obtain a pectin concentration of 1.51 g/l and a cell growth rate of 1.87 times.

### Examples 6 to 9

The culture was carried out in the same manner as in Example 5 except for a medium amount in Example 5 changes 60 ml, 80 ml, 100 ml and 120 ml to obtain pectins respectively. The results were shown in Table 3.

**[Table 3]**

| Example Nos. | 5 | 6 | 7 | 8 | 9 |
|---|---|---|---|---|---|
| Medium amount (ml) | 40 | 60 | 80 | 100 | 120 |
| Pectin concentration (g/l) | 1.51 | 1.24 | 0.86 | 0.66 | 0.51 |
| Growth rate (times) | 1.87 | 1.80 | 1.53 | 1.30 | 1.27 |

### Example 10

A seedling of Nigella sativa was used to induce a callus in the same manner as in Example 1, repeatedly cultured three times, whereby a stably proliferating cell line was obtained. The obtained cell was suspended in a liquid medium of the composition in Table 1 and cultured under the same conditions as in Example 7. The culture broth was analyzed to obtain a pectin concentration of 1.60 g/l and a cell growth rate of 18.6 times.

### Example 11

40 ml of 0.5M ethylenediaminetetracetic acid (pH 8) was added to 2L (5 flasks) of a solution in which a solid content such as cells or the like was removed by filtering from the culture broth obtained in Example 3 for solubilizing the pectin in the solution, and then fine solid content was further filtered off and separated. 6 L of ethanol was added to the filtrate and mildly stirred to obtain a pectin as a precipitate. After refrigerating for one day, the precipitate was filtered off to separate and redissolved in 1 L of water to obtain a pectin solution. The insoluble component was removed from the solution by filtering and freeze-dried to obtain 3.2 g of a pectin as a white powder.

### Example 12

(1) Preparation of a calibration curve
polyethylene glycol (molecular weight: 2 x 10⁶, a reagent of Wako Pure Chemical Industries, Ltd.) was dissolved in water such that its concentration became 1 g/l, and measured by the gel filtration chromatography under the following conditions.

| | |
|---|---|
| Column: | TSKgelG5000PWXL, a product of Tosoh Corporation |
| Mobile phase: | 0.1M sodium nitrate aqueous solution |
| Flow rate: | 0.5 ml/min |
| Temperature: | 40°C |
| Sample concentration: | aqueous solution of 1 g/l concentration |
| Sample input amount: | 10 µl |
| Detector: | Differential refractive index detector (RID-10A, a product of Shimadzu Corporation) |

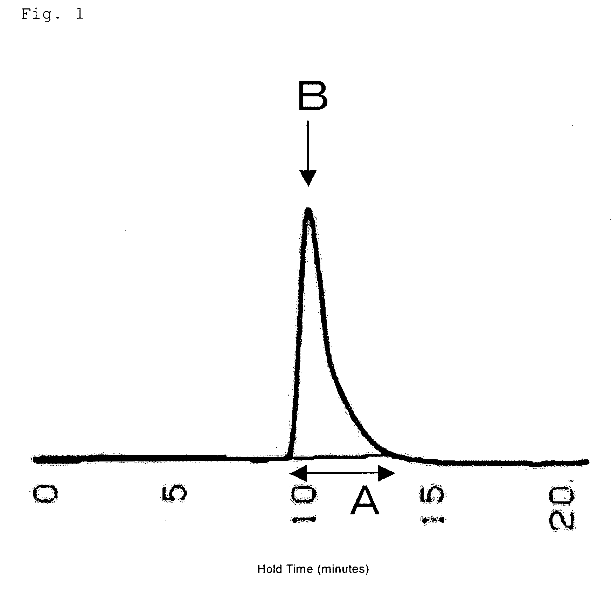

From the obtained distribution curve, the hold time indicating a peak was 10.8 minutes. Similarly, polyethylene oxide (molecular weight: 1 x 10⁶, 5 x 10⁵, 2 x 10⁵, a reagent of GL Science Co., Ltd.) was measured by the gel filtration chromatography and the hold times indicating peaks were 11.3 minutes, 11.8 minutes, and 13.2 minutes respectively. The relationship between the molecular weight and hold time was plotted to prepare a calibration curve.
(2) Measurement of a molecular weight of a pectin
The pectin obtained in Example 11 was dissolved in water such that its concentration became 1 g/l, and measured by the gel filtration chromatography under the above conditions. The obtained distribution curve showed that the hold time was in the range of from 9.1 to 13.2 minutes and the hold time indicating a peak was 10.8 minutes. From the calibration curve, the pectin obtained in Example 11 had the molecular weight in the range of 2 x 10⁵ to 1 x 10⁷ and the peak molecular weight of 2.0 x 10⁶ (Fig. 1).

### Test Example 1

The pectin of the present invention obtained in Example 11 was examined for its capability of maintaining moisture by the paper disc method. That is, an aqueous solution of the pectin of the present invention having a concentration of 1 g/l was prepared, 10 µl of a sample was immersed in a filter paper having a diameter of 8 mm and put under the conditions of a temperature of 25° and a relative humidity of 20% for measuring the weight change (residual rate of moisture) with time. As a comparison, water, an aqueous solution of sodium hyaluronate with a concentration 1 g/l (range of molecular weight of from 1 x 10⁵ to 5 x 10⁶, molecular weight peak of 1.7 x 10⁶, a product of Wako Pure Chemical Industries, Ltd.) and an aqueous solution of a commercial pectin with a concentration of 1 g/l (citrus-derived pectin, range of molecular weight of from 1 x 10⁴ to 8 x 10⁵, molecular weight peak of 1.0 x 10⁵, a product of Tokyo Kasei Kogyo Co., Ltd.) were used. The results were shown in Table 4. Since the pectin of the present invention had higher residual rate of moisture as compared with the comparison, it was found that the pectin had a high capability of maintaining moisture.

**[Table 4]**

| Time (minute) | Residual rate of moisture (%) | | | |
|---|---|---|---|---|
| | Water | Commercial pectin | Sodium hyaluronate | Pectin of the present invention |
| 0 | 100.0 | 100.0 | 100.0 | 100.0 |
| 2.5 | 87.1 | 87.7 | 90.1 | 91.4 |
| 5 | 74.2 | 76.9 | 79.2 | 81.6 |
| 7.5 | 62.4 | 65.3 | 69.3 | 72.0 |
| 10 | 51.5 | 54.7 | 59.4 | 62.2 |

### Test Example 2

An aqueous solution of the pectin having a concentration of 1 g/l of the present invention obtained in Example 11 was prepared and a test on water holding capacity for corneous stratum was carried out using a skin surface hygrometer, SKICON-200 (IBS Ltd.). The test method comprises adding 20 µl of the sample dropwise to the skin of the inner part on the shoulder under an environment of a temperature of 25° and a relative humidity of 20%, wiping it after 10 seconds, and measuring moisture content on the keratinous layer of the epidermic by the change in the electrical conductivity. As a comparison, water, an aqueous solution of sodium hyaluronate with a concentration 1 g/l and an aqueous solution of a commercial pectin with a concentration of 1 g/l used in Test Example 1 were used. The results were shown in Fig. 1. It was found that the pectin of the present invention exhibited higher capability of maintaining moisture on the skin than the commercial pectin and sodium hyaluronate, and skin penetrating property and affinity to the skin were excellent because of high initial moisture content.

### Test Example 3

An aqueous solution of the pectin having a concentration of 1 g/l of the present invention obtained in Example 11 was prepared and the viscosity at a stress of 1 Pa was measured at a temperature of 35°C using a rheometer (REOLOGICA Instruments). As a comparison, glycerin (Wako Pure Chemical Industries, Ltd.) and an aqueous solution of sodium hyaluronate (Wako Pure Chemical Industries, Ltd.) with a concentration of 1 g/l were used. The results were shown in Table 5. The pectin of the present invention exhibited higher viscosity than glycerin, but less viscosity than sodium hyaluronate. This can give a feeling with less stickiness to use it while maintaining a moist feeling and smooth feeling as a cosmetic material.

**[Table 5]**

| | Viscosity (mPa·s) |
|---|---|
| Pectin of the present invention | 11.8 |
| Sodium hyaluronate | 44.6 |
| Glycerin | 2.5 |

### Preparation Example 1

0.1 weight % of the pectin obtained in Example 11, 2.5 weight % of 1,3-butylene glycol, 0.5 weight % of glycerol (86%), 0.5 weight % of polyoxyethylene hydrogenated castor oil, 0.05 weight % of lactic acid, 0.7 weight % of sodium lactate, 7.0 weight % of ethanol, 0.1 weight % of methyl p-hydroxybenzoate, 0.05 weight % of perfume, and 88.5 weight % of purified water were prescribed to prepare a toilet water.

### Preparation Example 2

0.2 weight % of the pectin obtained in Example 11, 4.0 weight % of liquid paraffin, 4.0 weight % of squalane, 0.5 weight % of catanol, 1.5 weight % of stearic acid, 1.0 weight % of sorbitan monooleate, 1.0 weight % of polyoxyethylene sorbitan monooleate, 0.5 weight % of glycerol monostearate, 0.2 weight % of ethyl p-hydroxybenzoate, 3.0 weight % of glycerol (86%), 5.0 weight % of 1,3-butylene glycol, 0.05 weight % of perfume, and 79.05 weight % of purified water were prescribed to prepare a milky lotion.

### Preparation Example 3

0.5 weight % of the pectin obtained in Example 11, 8.0 weight % of vaseline, 2.0 weight % of lanolin, 20.0 weight % of squalane, 5.0 weight % of catanol, 2.0 weight % of glycerol monostearate, 2.0 weight % of polyoxyethylene sorbitan monooleate, 0.2 weight % of ethyl p-hydroxybenzoate, 5.0 weight % of glycerol (86%), 5.0 weight % of 1,3-butylene glycol, 0.1 weight % of perfume, and 50.2 weight % of purified water were prescribed to prepare a cream.

### Preparation Example 4

0.3 weight % of the pectin obtained by treating the culture broth obtained in Example 7 in the same manner as in Example 11, 18.0 weight % of polyvinyl alcohol, 2.0 weight % of polyethylene glycol, 5.0 weight % of 1,3-butylene glycol, 8.0 weight % of ethanol, 0.1 weight % of methyl p-hydroxybenzoate, 0.05 weight % of perfume, and 66.55 weight % of purified water were prescribed to prepare a pack.

### Preparation Example 5

1.0 weight % of the pectin obtained by treating the culture broth obtained in Example 10 in the same manner as in Example 11, 20.0 weight % of 1,3-butylene glycol, 15.0 weight % of glycerol (86%), 5.0 weight % of polyethylene glycol, 0.1 weight % of polyoxyethylene hexadecyl ether, 0.05 weight % of citric acid, 0.5 weight % of sodium citrate, 0.2 weight % of methyl p-hydroxybenzoate, 0.1 weight % of perfume, and 58.05 weight % of purified water were prescribed to prepare an essence.

### Preparation Example 6

0.3 weight % of the pectin obtained by treating the culture broth obtained in Example 7 in the same manner as in Example 11, 30.0 weight % of talc, 25.0 weight % of sericite, 10.0 weight % of titanium oxide, 7.0 weight % of modified silicon, 4.0 weight % of polyethylene powder, 2.0 weight % of squalane, 0.1 weight % of methyl p-hydroxybenzoate, 0.05 weight % of perfume, and 21.55 weight % of purified water were prescribed to prepare a foundation.

### Preparation Example 7

0.3 weight % of the pectin obtained by treating the culture broth obtained in Example 10 in the same manner as in Example 11, 45.0 weight % of modified silicon, 20.0 weight % of candelilla wax, 10.0 weight % of paraffin wax, 10.0 weight % of Carbana wax, 4.0 weight % of isobutyl isopalmitate, 3.7 weight % of diisostearyl malate, 2.0 weight % of isononyl isononanoate, 2.0 weight % of red 202, 1.0 weight % of red 101, 1.0 weight % of titanium oxide, and 1.0 weight % of yellow 4 were prescribed to prepare a rouge.

As described in Examples, it is possible to provide a pectin having a high molecular weight by the plant cell culture in the present invention. The obtained pectin having a high molecular weight has a high capability of maintaining moisture and low viscosity. Thus, the pectin can be used as a cosmetic material, in particular, as a moisturizer.

## Claims

1. A pectin comprising a galacturonic acid of from 70 to 90 mole % as a constituent sugar, and wherein the molecular weight measured by the gel filtration chromatography is in the range of 1 x 10⁴ to 5 x 10⁷ and the peak molecular weight is in the range of 2 x 10⁵ to 1 x 10⁷.

2. The pectin according to claim 1, wherein the mole ratio of the constituent sugars of a galacturonic acid : arabinose : galactose : glucuronic acid : glucose : xylose : rhamnose : mannose : fructose is 7 to 9 : 0.4 to 0.7 : 0.4 to 0.6 : 0.1 to 0.6 : 0.05 to 0.5 : 0.04 to 0.4 : 0 to 0.1 : 0 to 0.08 : 0 to 0.05.

3. The pectin according to claim 1 or 2, wherein the pectin is separated and recovered from the culture or the culture broth obtained by culturing a Nigella plant cell.

4. The pectin according to claim 3, wherein the Nigella plant is Nigella damascena or Nigella sativa.

5. Use of the pectin as described in any one of claims 1 to 4 as a moisturizer.

6. A cosmetic material containing the pectin as described in any one of claims 1 to 4.

7. A process for preparing the pectin as described in any one of claims 1 to 4, which comprises separating and recovering the pectin as described in any one of claims 1 to 4 from the culture or the culture broth obtained by culturing the Nigella plant cell.
